Europäisches Patentamt

European Patent Office

Office européen des brevets

(11) Publication number:

**0 058 686**

**B1**

(12) **EUROPEAN PATENT SPECIFICATION**

(45) Date of publication of patent specification: **26.02.86**

(21) Application number: **81902347.4**

(22) Date of filing: **12.08.81**

(86) International application number:
**PCT/US81/01081**

(87) International publication number:
**WO 82/00659 04.03.82 Gazette 82/07**

(51) Int. Cl.⁴: **C 12 N 9/88, C 12 N 1/20**

(54) PROCESS FOR PRODUCING HEPARINASE.

(30) Priority: **25.08.80 US 180780**

(43) Date of publication of application:
**01.09.82 Bulletin 82/35**

(45) Publication of the grant of the patent:
**26.02.86 Bulletin 86/09**

(84) Designated Contracting States:
**AT CH DE FR GB LI LU NL SE**

(56) References cited:
**US-A-3 549 500**

**The Journal of Biological Chemistry Vol. 245, No. 22, issued 25 November 1976, Baltimore, Maryland, U.S.A. Hovingh et al "The Enzymatic Degradation of Heparin and Heparitin Sulfate", Pages 6170-6175**

**The file contains technical information submitted after the application was filed and not included in this specification**

(73) Proprietor: **LANGER, Robert, J.**
**46 Greenville Street**
**Somerville, MA 02143 (US)**
(73) Proprietor: **LINHARDT, Robert**
**98 Elm Street**
**Somerville, MA 02144 (US)**
(73) Proprietor: **COONEY, Charles L.**
**35 Chestnut Place**
**Brookline, MA 02146 (US)**
(73) Proprietor: **GALLIHER, Parrish M.**
**144 Cherry Street**
**West Newton, MA 02165 (US)**

(72) Inventor: **LANGER, Robert, J.**
**46 Greenville Street**
**Somerville, MA 02143 (US)**
Inventor: **LINHARDT, Robert**
**98 Elm Street**
**Somerville, MA 02144 (US)**
Inventor: **COONEY, Charles L.**
**35 Chestnut Place**
**Brookline, MA 02146 (US)**
Inventor: **GALLIHER, Parrish M.**
**144 Cherry Street**
**West Newton, MA 02165 (US)**

Courier Press, Leamington Spa, England.

**0 058 686**

Representative: **Holdcroft, James Gerald, Dr. et al**
**Graham Watt & Co. Riverhead**
**Sevenoaks Kent TN13 2BN (GB)**

References cited:
**Methods in Enzymology, Colowick et al, editors, Vol. 28, 1972 Academic Press, New York, N.Y., U.S.A: Section 123 "Heparinase and Heparitinase from Flavobacteria" by Linker et al, pages 902-911**

**CHEMICAL ABSTRACTS, vol. 91, no. 25, December 17, 1979, page 520, abstract no. 209379d COLUMBUS OHIO (US)**

**CHEMICAL ABSTRACTS, vol. 79, no. 25, December 24, 1973, page 115, abstract no. 143875f COLUMBUS OHIO (US)**

**CARBOHYDRATE RESEARCH, vol. 88, no. 2, Elsevier Scientific Publishing Company, 1981 AMSTERDAM (NL) N. OTOTANI et al.: "Purification of heparinase and heparitinase by affinity chromatography on glycos-aminoglycan-bound AH-sepharose 4B", pages 291-303**

**CHEMICAL ABSTRACTS, vol. 94, no. 19, May 11, 1981, page 499, abstract no. 154929n COLUMBUS OHIO (US)**

Description

This invention relates to a process for producing heparinase utilizing a defined medium followed by a purification step.

Heparinase is an enzyme presently used in assays for heparin. Presently, heparinase is produced from *Flavobacterium heparinum* utilizing protein as the carbon, and nitrogen source and a phosphate source (see for example "Methods in Enzymology", Colowick *et al.,* editors, Volume 28, 1972, Academic Press, New York, N.Y. U.S.A., Section 123, "Heparinase and Heparitinase from Flavobacteria", by Linker *et al.,* pages 902 to 911). In addition, presently available processes for producing heparinase provide a volumetric productivity of heparinase of about 1.8 mg heparin degraded/liter culture—hour. Furthermore, these prior processes require six stages of production including: (1) 72 hour fermentation; (2) Harvest by centrifugation; (3) 24 hour fermentation; (4) Harvest by centrifugation; (5) 17. hour incubation and (6) Harvest and sonication. In present processes, the crude enzyme produced by the bacterium is isolated by a step which includes passage of the crude enzyme through a column of hydroxylapatite $(Ca_x)$ $(PO_4)y$). It has been found that the heparinase binds more tightly to the hydroxylapatite than 90% of the protein present in *Flavobacterium heparinum*. Thus, a hydroxylapatite column can provide from 10—100 fold enzyme enrichment when the protein is eluted from the column at high eluent salt concentration. However, hydroxylapatite is fragile and has poor flow characteristics in large columns and thus is not amenable to purification of much more than 1—10 grams of crude enzyme. Furthermore, each such purification requires nearly a week's time.

Accordingly, it would be desirable to provide a process for purifying heparinase which is not limited by the materials and method utilized in purifying heparinase and which requires far less time than required by present available processes. In addition, it would be desirable to provide such a process which drastically increases the volumetric productivity of heparinase.

In an embodiment of this invention by way of example, a process for producing heparinase is provided wherein the growth medium is a chemically defined medium comprising a carbon source, at least two amino acids, a phosphate source, a magnesium source, a heparinase inducer and trace salts in the absence of a chemically non-defined substance normally present in growth medium including a protein digest or yeast extract. The product is purified in a batch process wherein the cell pellet is disrupted such as sonically, homogenization, enzyme treatment, osmotic shock. The resultant extract then is mixed with hydroxylapatite followed by isolation of the heparinase bound to the hydroxylapatite utilizing an eluent comprised of phosphate buffer and sodium chloride in a stepwise fashion of increasing concentration of salt element such as sodium chloride, sodium phosphate, sodium acetate, the corresponding potassium salts or mixture thereof. A typical elution schedule is as follows:

1) 0.01M Sodium Phosphate pH 6.8
2) 0.02M Sodium Phosphate .03 Sodium Chloride pH 6.8
3) 0.04 Sodium Phosphate .06M Sodium Chloride pH 6.8
4) 0.055M Sodium Phosphate .09M Sodium Chloride pH 6.8
5) 0.07M Sodium Phosphate .125M Sodium Chloride pH 6.8
6) 0.085M Sodium Phosphate .16M Sodium Chloride pH 6.8
7) 0.10M Sodium Phosphate .19M Sodium Chloride pH 6.8

By utilizing this procedure, heparinase can be recovered from the 5th wash in as little a time as 8 hour with a specific activity of 80 mg heparin degraded/mg protein-hour. Accordingly, substantial efficiency and productivity for producing heparinase is provided as compared to present available processes.

The invention provides a process for producing purified heparinase by growing *Flavobacterium heparinum* (or a mutant thereof capable of producing heparinase) in a growth medium until heparinase is formed, characterised in that the growth medium is free of protein and yeast extract and comprises a carbon source, a phosphate source, a magnesium source, at least two amino acids, and trace salts; and in that an extract of the *Flavobacterium heparinum* (or mutant thereof capable of producing heparinase) containing heparinase is intimately admixed with between 5 and 15 weight per cent hydroxylapatite based upon the weight of the mixture to bind said heparinase to said hydroxylapatite, said hydroxylapatite containing bound heparinase being recovered; and said bound heparinase is recovered by elution.

Description of specific embodiments

The strain of bacterium utilized comprises *Flavobacterium heparinum* such as *Flavobacterium heparinum* ATCC 13125 or a mutant form of this bacterium which is capable of producing heparinase.

The bacterium is grown in a chemically defined growth medium, i.e., a growth medium devoid of proteins, yeast extract or complex nutrients which are difficult to characterize and/or which vary in characteristics depending upon their source. Examples of carbon sources which can be utilized in the growth medium include glucose, glycerol, maltose or heparin at concentrations, for example of between 0 g/l and 20 g/l, usually between 5 g/l and 10 g/l. It is preferred to utilize glucose as the carbon source at a concentration of between 5 g/l and 10 g/l because of low cost.

The growth medium contains a source of phosphate; examples include monobasic or dibasic potassium phosphate, sodium mono or dibasic phosphate, ammonium phosphate or mixtures thereof. The growhm medium includes at least two amino acids and may include an additional source of nitrogen; examples of which include ammonium sulfate or heparin; and a source of magnesium examples of which include magnesium sulfate, magnesium chloride or magnesium phosphate. The growth medium may include a heparinase inducer examples of which include sodium heparin, heparin monosulfate, hyaluronic acid, maltose, N-acetyl D-glucosamine or the like. Certain mutants of Flavobacterium heparinum need not require a heparinase inducer. A typical growth medium contains glucose, ammonium sulfate, and a mixture of potassium monobasic phosphate and sodium dibasic phosphate, magnesium sulfate, trace salts, L-methionine and L-histidine and the heparinase inducer. The ammonium sulfate can comprise between 0.5 g/l and 10 g/l, preferably 2 g/l; the mixture of phosphates can comprise between 1 g/l and 12 g/l, preferably 5 g/l; the magnesium sulfate comprises between 0.1 g/l and 1 g/l preferably 0.5 g/l; and the heparinase inducer comprises between .05 g/l and 10 g/l preferably 1.0 g/l. Trace salts were comprised of $Na_2MoO_4$, $CoCl_2$, $MnSO_4$, $CuSO_4$, $FeSO_4$, $CaCl_2$ all at $1 \times 10^{-4}$M; total L-histidine and L-methionine at 0.2—1 g/l (e.g. 0.2—0.5 g/l).

The pH of the medium generally is maintained between 6 and 8, preferably about 7. It is preferred to control the pH at about 7 during the course of the fermentation by the addition of ammonium hydroxide or sodium hydroxide. Sterile air is sparged into the fermentor at a rate sufficient to meet the needs of the bacterium and typically between .25 VVM and 5.0 VVM. The dissolved oxygen is set between 0 and 100% typically at 50%. The growth medium is maintained at a temperature between 15°C and 32°C, preferably between 22°C and 25°C. Optionally, the growth medium can contain an antifoaming agent such as P-2000 manufactured by Dow Chemical Company at a concentration between 0.1 ml/l and 1 ml/l to control foaming.

The crude product then is purified by admixture in a batch mode with hydroxylapatite wherein the hydroxylapatite comprises between 5 weight percent and 15 weight percent of the mixture. The mixture is stirred gently for a period of time between 1 minute and 5 minutes at a temperature between 0°C and 10°C in order to selectively immobilize the heparinase on the hydroxylapatite. The immobilized heparinase then is removed from the hydroxylapatite by wash with increasing salt concentrations such as with sodium chloride and sodium phosphate at pH 6.8. Generally, the enriched eluent fractions are those containing about 0.07M sodium phosphate and 0.125M sodium chloride or between 0.055M sodium phosphate, 0.09M sodium chloride and 0.085 M sodium phosphate, 0.16M sodium chloride and at pH 6.8.

The following examples illustrate the present invention and are not intended to limit the same.

Example I

Flavobacterium heparinum ATCC 13125 was grown in a 14 liter fermentor at a 23°C, pH 7.0 (controlled by ammonium hydroxide addition) and aerated at a rate of 0.5 VVM with dissolved oxygen maintained at 50% of air saturation, 10 liters of a culture medium comprised of 10 g/l glucose, 2 g/l $(NH_4)_2SO_4$, 2.5 g/l $KH_2PO_4$, 2.5 g/l $NaH_2PO_4$ 0.5 g/l $MgSO_4 \cdot 7H_2O$, 1.0 g/l sodium heparin, 0.1 g/l P-2000 anti-foaming agent, 0.5 g/l L-histidine, 0.5 g/l L-methionine trace salts $10^{-4}$M. After about 25 hours of growth, the cells were harvested, centrifuged at 12,000×G, resuspended in 0.01M phosphate buffer pH 6.8, sonicated to release 90% of protein. To this 6.5 g protamine sulfate was added, after 1 h at 4°C was centrifuged at 12,000×G. This supernate was diluted with $H_2O$ to 2 liters then were admixed with 250 g hydroxylapatite at a temperature at 4°C and at a pH of 6.8. The mixture was stirred for about 5 minutes in order to substantially completely bind heparinase to the hydroxylapatite. The hydroxylapatite then was isolated from the mixture by centrifugation at 1000×G and the supernate was decanted from it. The heparinase was eluted by washing with sodium chloride and sodium phosphate washes (pH 6.8) from 0.05M Na phosphate to 0.1M Na phosphate, 0.19M NaCl and recovering the eluent. The heparinase was concentrated in the eluent comprising 0.07M Na phosphate and 0.125M sodium chloride.

**Claims**

1. A process for producing purified heparinase by growing *Flavobacterium heparinum* (or a mutant thereof capable of producing heparinase) in a growth medium until heparinase is formed, characterised in that the growth medium is free of protein and yeast extract and comprises a carbon source, a phosphate source, a magnesium source, at least two amino acids, and trace salts; and in that an extract of the *Flavobacterium heparinum* (or mutant thereof capable of producing heparinase) containing heparinase is intimately admixed with between 5 and 15 weight per cent hydroxylapatite based upon the weight of the mixture to bind said heparinase to said hydroxylapatite, said hydroxylapatite containing bound heparinase being recovered; and said bound heparinase is recovered by elution.

2. A process according to claim 1, wherein the growth medium also comprises a source of nitrogen additional to said amino acids.

3. A process according to claim 2, wherein the carbon source is selected from any of glucose, glycerol and maltose; the phosphate source is selected from any of monobasic or dibasic sodium or potassium phosphate, and ammonium phosphate; the additional nitrogen source comprises ammonium sulfate; the magnesium source is selected from any of magnesium sulfate, chloride and phosphate; and said at least two amino acids are L-methionine and L-histidine.

4. A process according to claim 3, wherein the growth medium comprises up to 20 grams per litre (e.g. between 5 and 10 grams per litre) glucose; between 1 and 12 grams per litre (e.g. 5 grams per litre) of a mixture of potassium and sodium phosphates; between 0.5 and 10 grams per litre (e.g 2 grams per litre) ammonium sulfate; between 0.1 and 1 gram per litre (e.g. 0.5 grams per litre) magnesium sulfate (MgSO$_4 \cdot$ 7H$_2$O); and 0.2—1 gram per litre (e.g. 0.2—0.5 grams per litre) of a mixture of L-methionine and L-histidine.

5. A process according to any one of the preceding claims, wherein the growth medium comprises a heparinase inducer.

6. A process according to claim 5, wherein the heparinase inducer is selected from any of sodium heparin, heparin monosulfate, hyalauronic acid, and N-acetyl-D-glucosamine.

7. A process according to claim 6, wherein the growth medium comprises between .05 and 10 grams per litre (e.g. 1 gram per litre) sodium heparin.

8. A process according to any one of the preceding claims, wherein elution is effected with sodium phosphate and sodium chloride.

## Patentansprüche

1. Verfahren zum Herstellen gereinigter Heparinase durch Züchtung von Flavobacterium Heparinum (oder einer heparinasebildungsfähigen Mutante von diesem) in einem Wachstumsmedium, bis Heparinase gebildet ist, dadurch gekennzeichnet, daß das Wachstumsmedium frei von Protein und Hefeextrakt ist und eine Kohlenstoffquelle, eine Phosphatquelle, eine Magnesiumquelle, zumindest zwei Aminosäuren und Spurensalze umfaßt und daß ein heparinasehaltiger Extrakt des Flavobacteriums Heparinum (oder dessen heparinasebildungsfähiger Mutante) mit 5 bis 15 Gewichtsprozent Hydroxylapatit, basierend auf dem Gewicht der Mischung, innig gemischt wird, um die Heparinase an den Hydroxylapatit zu binden, der gebunde Heparinase enthaltende Hydroxylapatit wiedergewonnen wird und die gebundene Heparinase durch Elution wiedergewonnen wird.

2. Verfahren nach Anspruch 1, dadurch gekennzeichnet, daß das Wachstumsmedium auch eine Stickstoffquelle zusätzlich zu den Aminosäuren umfaßt.

3. Verfahren nach Anspruch 2, dadurch gekennzeichnet, daß die Kohlenstoffquelle aus einem der Stoffe Glucose, Glycerol und Maltose ausgewählt wird, die Phosphatquelle aus einem der Stoffe ein- oder zweibasisches Natrium- oder Kaliumphosphat und Ammoniumphosphat ausgewählt wird, die zusätzliche Stickstoffquelle aus Ammoniumsulfat besteht, die Magnesiumquelle aus einem der Stoffe Magnesiumsulfat, -chlorid und -phosphat ausgewählt wird und die beiden zumindest vorgesehenen Aminosäuren L-Methionin und L-Histidin sind.

4. Verfahren nach Anspruch 3, dadurch gekennzeichnet, daß das Wachstumsmedium bis zu 20 Gram pro Liter (z.B. 5 bis 10 Gramm pro Liter) Glucose, 1 bis 12 Gramm pro Liter (z.B. 5 Gramm pro Liter) einer Mischung von Kalium- und Natriumphosphaten, 0,5 bis 10 Gram pro Liter (z.B. 2 Gramm pro Liter) Ammoniumsulfat, 0,1 bis 1 Gramm pro Liter (z.B. 0,5 Gramm pro Liter) Magnesiumsulfat (MgSO$_4 \cdot$ 7H$_2$O) und 0,2 bis 1 Gramm pro Liter (z.B. 0,2 bis 0,5 Gramm pro Liter) einer Mischung von L-Methionin und L-Histidin umfaßt.

5. Verfahren nach einem der vorhergehenden Ansprüche, dadurch gekennzeichnet, daß das Wachstumsmedium ein Heparinase-Induziermittel umfaßt.

6. Verfahren nach Anspruch 5, dadurch gekennzeichnet, daß das Heparinase-Induziermittel aus einem der Stoffe Natriumheparin, Heparinmonosulfat, Hyaluronsäure und N-Acetyl-D-Glucosamin ausgewählt wird.

7. Verfahren nach Anspruch 6, dadurch gekennzeichnet, daß das Wachstumsmedium 0,5 bis 10 Gramm pro Liter (z.B. 1 Gramm pro Liter) Natriumheparin umfaßt.

8. Verfahren nach einem der vorhergehenden Ansprüche, dadurch gekennzeichnet, daß die Elution mit Natriumphosphat und Natriumchlorid durchgeführt wird.

## Revendications

1. Un procédé pour produire de l'héparinase purifiée en cultivant la bactérie *Flavobacterium heparinum* (ou un mutant de celle-ci capable de produire l'héparinase) dans un milieu de culture jusqu'à ce que l'héparinase soit formée, caractérisé en ce que le milieu de culture ne comporte pas de protéine et d'extrait de levure et comprend une source de carbone, une source de phosphate, une source de magnésium, au moins deux acides aminés et des traces de sels; et en ce que l'extrait de *Flavobacterium heparinum* (ou un mutant de celle-ci capable de produire de l'héparinase) contenant l'héparinase est mélangé intimement avec entre 5 et 15% en poids d'hydroxylapatite basés sur le poids du mélange pour lier cette héparinase à cette hydroxylapatite, cette hydroxylapatite contenant l'héparinase liée étant récupérée; et cette héparinase liée étant récupérée sous élution.

2. Procédé selon la revendication 1, au cours duquel le milieu de culture comprend également une source d'azote en plus de ces acides aminés.

3. Procédé selon la revendication 2, au cours duquel la source de carbone est choisie parmi l'un quelconque des glucose, glycérol et maltose; la source de phosphate est choisie parmi l'un quelconque des phosphates monobasiques ou dibasiques de sodium ou de potassium, et phosphate d'ammonium; la source d'azote supplémentaire comprend du sulfate d'ammonium; la source de magnésium est choisie parmi l'un quelconque des sulfates, chlorures et phosphates de magnésium; et ces au moins deux acides aminés sont la L-méthionine et L-histidine.

4. Procédé selon la revendication 3, au cours duquel le milieu de culture comprend jusqu'à 20 g/l (par exemple entre 5 et 10 g/l) de glucose; entre 1 et 12 g/l (par exemple 5 g/l) d'un mélange de phosphate de potassium et de sodium; entre 0,5 et 10 g/l (par exemple 2 g/l) de sulfate d'ammonium; entre 0,1 et 1 g/l (par exemple 0,5 g/l) de sulfate de magnésium (MgSO$_4 \cdot$ 7H$_2$O); et 0,2—1 g/l (par exemple 0,2—0,5 g/l) d'un mélange de L-méthionine et L-histidine.

5. Procédé selon l'une quelconque des revendications précédentes dans lequel le milieu de culture comprend un inducteur d'héparinase.

6. Procédé selon la revendication 5, dans lequel l'inducteur d'héparinase est choisi parmi l'un quelconque des héparine sodique, monosulfate d'héparine, acide hyaluronique et N-acétyl-D-glucosamine.

7. Procédé selon la revendication 6, dans lequel le milieu de culture comprend entre 0,05 et 10 g/l (par exemple 1 g/l) d'héparine sodique.

8. Procédé selon l'une quelconque des revendications précédentes dans lequel l'élution est effectuée avec du phosphate de sodium et du chlorure de sodium.